# EUROPÄISCHE PATENTSCHRIFT

(11) **EP 1 894 593 B1**
(45) Veröffentlichungstag und Bekanntmachung des Hinweises auf die Patenterteilung: **20.04.2011**
(21) Anmeldenummer: 07017119.4
(22) Anmeldetag: 31.08.2007
(51) Int. Cl.: A61M 25/00, A61M 25/04, A61M 29/00, A61M 27/00

(54) **Vorrichtung zur Kanülierung eines Blut-führenden Gefässes**
Device for introducing a cannula into a blood vessel
Dispositif de canulage d'un vaisseau sanguin

(30) Priorität: 01.09.2006 DE 102006042639; 24.07.2007 DE 102007037050
(43) Veröffentlichungstag der Anmeldung: 05.03.2008
(73) Patentinhaber: NovaLung GmbH, 72379 Hechingen (DE)
(72) Erfinder: Cattaneo, Giorgio, 72074 Tübingen (DE); Spranger, Martin, 72119 Entringen (DE)
(74) Vertreter: Graf von Stosch, Andreas

(56) Entgegenhaltungen:
- EP-A- 0 301 854
- WO-A-2005/002454
- FR-A- 2 220 283
- US-A1- 2004 082 906
- US-A1- 2005 027 245
- US-A1- 2005 107 820
- US-B1- 6 270 490
- US-B1- 6 673 042

## Beschreibung

Die vorliegende Erfindung betrifft eine Vorrichtung zur Kanülierung eines Hohlorgans, mit einer Kanüle, die ein proximales Ende, ein distales Ende und einen zwischen dem proximalen und dem distalen Ende gelegenen Zwischenabschnitt aufweist, wobei die Kanüle nach Einbringung in das Gefäß in Fluidverbindung mit dem Gefäß steht.

Derartige Vorrichtungen, die eine Kanüle aufweisen, werden im chirurgischen und medizinischen Bereich vielfältig eingesetzt, wie bspw. zur Kanülierung von arteriellen oder venösen Blut-führenden Gefäßen. Eine typische Kanüle stellt ein Röhrchen mit einem vorwiegend festgesetzten Durchmesser dar, welches ein Chirurg oder Arzt dazu einsetzt, um Blut abzunehmen, bzw. um einen Blutdurchgang zwischen dem Blutkreislauf eines Patienten und einem extrakorporalen System zu schaffen. So kann ein extrakorporaler Kreislauf für bspw. eine extrakorporale Blutoxygenierung durch eine Blutpumpe aufrechterhalten werden, oder aber, unter Ausnützung des körpereigenen Blutdrucks, pumpenlos. Die extrakorporale Lungenunzerstützung (iLA, AVCO2R, pECLA, ECMO etc.) kommt bei Patienten mit akutem Lungenversagen zum Einsatz, bei welchen unter Vermeidung der Risiken der konventionellen Beatmung Kohlendioxid aus dem Blut entfernt und eine Sauerstoffanreicherung stattfindet. Das Sauerstoff-reiche Blut wird anschließend dem Patienten wieder zugeführt. Dabei werden zwei einzelne Kanülen oder eine Doppellumenkanüle in Periphergefäße eingebracht, wonach das Blut über ein kurzes Schlauchsystem durch eine sogenannte Membranlunge und zurück in den Kreislauf des Patienten fließt. Bei Anwendungen in der Intensivmedizin werden Kanülen beispielsweise durch ein Periphergefäß des Beines oder des Halses eingeführt. Dieses System hat den Vorteil, dass über Tage und sogar Wochen hinweg ein ausreichender Gasaustausch erreicht werden kann, wodurch bspw. aggressive Beatmungen und beatmungsinduzierte Schäden (VALI) vermieden werden können und sich die Lunge regenerieren kann.

Kanülen sind mit verschiedenen Dimensionen und Durchmessern in Abhängigkeit des Gefäßes, insbesondere dessen Größe, und Anwendung einsetzbar. Gewöhnlich besitzen flexible Katheter oder Kanülen ein festgesetztes Lumen und eine festgesetzte Größe hinsichtlich ihres Außendurchmessers. So muss beispielweise bei einem Herz-Lungen-Bypass ein großes Lumen bereitgestellt werden, um einen hinreichenden Fluss mit oxygeniertem Blut zu ermöglichen. Hierfür muss ein traditioneller Katheter oder eine Kanüle einen großen Durchmesser haben, was wiederum die Einführung der Kanüle/des Katheters in das Gefäß und insbesondere die Gewebedurchdringung erschwert und zu einem chirurgischen Vorgehen mit größeren Schädigungen des umliegenden Gewebes führen kann.

Bei einer Kanülierung von arteriellen Gefäßen, wird bei kleineren Kanülen aufgrund des kleineren Lumendurchmessers der Widerstand des Blutdurchflusses innerhalb der Kanüle erhöht, was eine Beschränkung der Durchflussrate zur Folge hat. Bei einem Einsatz von Blutpumpen werden hohe Durchflussraten erzwungen, wodurch die resultierenden hohen Schubspannungen in der Blutströmung eine Gefahr der Blutzellschädigung darstellen.

Größere Kanülen ermöglichen andererseits höhere Durchflussraten bei gleichzeitiger niedrigerer Belastung des Blutes. Andererseits besteht bei größeren Kanülen die Gefahr, dass das Blutgefäß nach Einbringung der Kanüle verschlossen wird und die physiologischen Strömungsverhältnisse in weiteren Teilen des Kreislaufs gestört werden. Hierbei kann bspw. bei einem Zugang in ein arterielles Gefäß die physiologische Durchblutung der nachgeschalteten Strombahn z.B. in Körperextremitäten oder andere Organe nachteilig verhindert werden. Durch diese Blockierung des Blutflusses kann es dort zu Ischämie kommen, einem Missverhältnis zwischen Sauerstoff- und Nährstoffbedarf und -versorgung mit konsequentem Absterben des betroffenen Gewebes. Ferner kann es bei der Kanülierung eines venösen Gefäßes zu einer Stauung des Blutrückflusses zum Herzen mit konsekutiver Organschädigung und Gerinnungsgefahr kommen.

Aufgrund der oben geschilderten Gefahren spielt daher die Bestimmung der Größe der Kanüle bei einem pumpenlosen extrakorporalen Kreislauf mit artero-venösem Anschluss ebenso wie bei einem venovenösen oder venoarteeriellen extrakorporalen Kreislauf mit Blutpumpe eine tragende Rolle. Wie oben erwähnt, ist die Blutflussrate in Abwesenheit einer Pumpe von den physiologischen Blutdruckverhältnissen im Kreislauf, von der Größe der Kanüle und dem Widerstand des extrakorporalen Systems (künstliche Lunge, Hämofilter, Schläuche etc.) abhängig.

Um größere Blutdurchflüsse zu erzielen, sind Kanülen mit einem großen Lumen erforderlich, was wiederum den oben genannten Nachteil der Ischämie oder venösen Blutstauungmit sich bringen kann.

Da im venösen Kreislaufbereich ein geringer Blutdruck herrscht, muss zur aktiven venösen Drainage für den extrakorporalen Kreislauf Druck aufgebaut werden, was durch den Einsatz einer Pumpe erreicht wird. Dabei kann es bei hohen Blutdurchflüssen durch die Kanüle zu einer Abnahme des Blutdrucks, und somit zu einem Unterdruck im Gefäß kommen. Dies kann zum Kollabieren des Gefäßes führen, da die Gefäßwände von dem außerhalb des Gefäßes herrschenden Drucks nach innen gedrückt, was zum Verschluss des Gefäßes und zur Unterbrechung der Blutströmung führt.

Daher spielen der Durchmesser und die Länge der venösen Kanüle in Abhängigkeit vom gewünschten Blutfluss auch bei der venösen Blutzufuhr in einen extrakorporalen Kreislauf mit veno-venösem oder venoarteriellem Anschluss eine tragende Rolle. Sie bestimmt z.B. auch das Ausmaß bzw. die Vermeidung des Bluttraumas durch Unterdruck bzw. resultierende übermäßige Scherkräfte

Im Stand der Technik sind Kanülen bekannt, die über ihre gesamte Länge im Lumen erweitert werden können, sowie Kanülen, die darüber hinaus einen Okklusionsballon an einem Ende aufweisen.

So ist aus der US 6,613,038 B2 eine Kanüle bekannt, die durch das Gewebe in einem unexpandierten Zustand mit einem kleinen Durchmesser eingeführt wird, und die dann durch Entfernen einer Hülle der Kanüle in ihrer gesamten Länge radial auswärts expandiert, sobald sie in das gewünschte Gefäß bzw. Gewebe eingeführt worden ist.

Ferner ist aus der US 6,679,871 eine Kanüle bekannt, welche entlang ihrem Lumen einen inflatierbaren Ballon aufweist, der nach Einbringen in ein Blutgefäß expandiert werden kann. Dieser Katheter kann ferner einen verschließenden Ballon aufweisen, der nach seiner Expansion das Gefäß komplett verschließt.

Jedes der Dokumente EP 030 1 854 (D1) und US 667 3 042 (D2) offenbart eine Vorrichtung zur Kanülierung gemäß dem Oberbegriff von Anspruch 1.

Die oben genannten Ausführungsformen der Kanülen sind jedoch nicht geeignet, die im Stand der Technik bekannten Probleme zu überwinden, da auch bei diesen Kanülen die Gefahr besteht, dass das Gefäß verschlossen wird und bei einem arteriellen Einsatz die Durchblutung der Körperextremitäten verhindert wird.

Daher ist es Aufgabe der vorliegenden Erfindung, eine Vorrichtung zu Kanülierung eines Hohlorgans bereitzustellen, mit der die im Stand der Technik beschriebenen Nachteile überwunden werden können.

Gemäß der vorliegenden Erfindung wird die Aufgabe durch eine Vorrichtung zur Kanülierung, wie in Anspruch 1 beansprucht gelöst.

Die der Erfindung zugrunde liegende Aufgabe wird auf diese Weise vollkommen gelöst.

Vorliegend wird mit "proximal" das Ende der Kanüle bezeichnet, das zum Anwender hin gerichtet ist und nach dem Einsatz der Kanüle außerhalb des Gefäßes zu liegen kommt.

Mit "distal" wird vorliegend das Ende der Kanüle bezeichnet, das vom Anwender weg zeigt, und in das Gefäß einzuführen ist.

Mit der Vorrichtung gemäß der Erfindung wird sichergestellt, dass nach Einführen der Kanüle in ein Gefäß zum einen die Herstellung des Blutdurchgangs zwischen Blutkreislauf und extrakorporalem System gewährleistet ist, und dass andererseits aber durch die Öffnungen in der expandierbaren Struktur gewährleistet ist, dass die stromabwärts der Kanülierungsstelle gelegen Körperteile eines Patienten weiterhin mit Blut versorgt werden. Ein Absterben des Gewebes bzw. Ischämien werden dadurch vorteilhaft vermieden.

Ferner wird bei einer Kanülierung eines venösen Gefäßes, insbesondere in ein peripheres Gefäß wie die Vena femoralis oder die Vena jugularis, durch die Öffnungen in der expandierbaren Struktur am distalen Ende gewährleistet, dass das Gefäßlumen in einem offenen Zustand gehalten und die Kollabierung des Gefäßes vermieden wird. Gleichzeitig wird das Ansaugen der Gefäßwand durch die Kanüle und des resultierende Verschließen der Kanüle verhindert, sodass dadurch sowohl der Blutfluss in dem Gefäß als auch der Blutfluss in der venösen Kanüle gesichert wird. Ferner besteht nicht die Gefahr einer Hämolyse und der damit einhergehende Beschädigung von Erythrozyten sowie einer Leukozytenaktivierung, welche durch Saugeffekte hervorgerufen werden kann.

Die erwähnten Vorteile werden dadurch gewährleistet, dass nach Einbringung der Kanüle in das Gefäß das distale Ende expandiert werden kann, wobei gleichzeitig die Öffnungen der expandierenden Struktur des distalen Endes freigegeben werden. Mit der Expansion kann sich darüber hinaus die expandierende Struktur des distalen Endes bspw. an die Gefäßwand des Blut-führenden Gefäßes anlegen, wodurch vorteilhafterweise ferner ein sicherer Sitz der Kanüle in dem Gefäß gewährleistet wird. Unabhängig davon, ob sich die expandierbare Struktur an die Gefäßwand anlegt oder nicht, wird mit der expandierbaren Struktur allgemein gewährleistet, dass zwischen Kanüle und Gefäßwand ein Abstand gewährleistet wird, der ein Verschließen des Gefäßes verhindert.

Um eine hinreichende Blutentnahme zu gewährleisten, weist die Kanüle darüber hinaus eine derartige Größe bzw. Durchmesser auf, dass sie nicht jeweils vollständig an den Gefäßwänden anliegt, sondern in ihrem Bereich, der sich vom proximalen Ende und über den Zwischenabschnitt erstreckt, jeweils einen größeren Durchmesser als das Gefäß besitzt. Das distale Ende weist vorzugsweise vor und während des Einführens der Kanüle einen noch kleineren Durchmesser auf als das proximale Ende und der Zwischenabschnitt, um einen möglichst schonenden Zugang zu dem Gefäß zu verschaffen.

Durch die expandierbare Struktur und den hierin vorgesehenen Öffnungen wird gewährleistet, dass ein Teil des Blutes an der Kanüle vorbei fließen kann, und somit in Körperpartien gelangt, die stromabwärts der Kanülierungsstelle liegen, so dass eine ausreichende Blutversorgung dieser Gefäße und insbesondere der Körperextremitäten gewährleistet ist. Auf der anderen Seite ist sichergestellt, dass eine hinreichende Menge Blutes im Körper über die Kanüle entnommen werden kann. Das dem Gefäß über die Kanüle entnommene Blut wird bspw. einem extrakorporalen Kreislauf zugeführt und - bei einem extrakorporalen Herz-Lungen-Kreislauf - bspw. über Membranoxygenatoren mit Sauerstoff angereichert und dem Körper wiederum über venöse oder arterielle Kanülen zugeführt. Ferner wird gewährleistet, dass bei einer venösen Kanülierung das Gefäß an oder nahe der Kanülierungsstelle nicht kollabiert und dadurch ein venöser Unterdruck und eine folgende Blutschädigung vermieden wird.

Mit der erfindungsgemäßen Kanüle wird eine Vorrichtung zur Kanülierung eines arteriellen oder venösen Blut-führenden Gefäßes bereitgestellt, die einen kleinen Widerstand zur Blutströmung und gleichzeitig einen physiologischen Rückfluss aus der Körperperipherie ermöglicht. Mit der vorliegenden Erfindung wird vermieden, dass die Kanüle zum Verschluss eines Gefäßes führt und damit die Blutströmung um die Kanüle beeinträchtigt wird.

Ferner ist bei der erfindungsgemäßen Vorrichtung von Vorteil, dass die Kanüle hinsichtlich ihrer Größe bzw. durchschnittlichem Durchmesser dem jeweils zu kanülierenden Gefäß angepasst werden kann, wodurch ein erhöhter Blutfluss innerhalb der Kanüle trotz beschränkter Einführungsgröße ermöglicht wird. Dem Fachmann wird klar sein, dass die Größe bzw. der Durchmesser der Kanüle von der Größe des Gefäßes abhängt, des kanüliert werden soll, und kann demnach in Abhängigkeit von Patient, gewünschtem Einsatzzweck und Gefäß variieren.

Während der Einführung durch die Gefäßwand wird dabei die expandierbare Struktur am distalen Ende der Vorrichtung in einem zusammengedrückten/Querschnittskomprimierten bzw. nicht-expandierten Zustand gehalten, und weist einen beschränkten Durchmesser auf. Nach Einbringen der Kanüle in das Gefäß kann das distale Ende mit der expandierbaren Struktur in den expandierten Zustand überführt werden, wodurch die expandierbare Struktur am distalen Ende in Kontakt mit der Gefäßwand kommen kann. Durch die durch die expandierbare Struktur ausgeübte Kraft in radialer Richtung wird das Gefäß offen gehalten, insbesondere durch die Gewährleistung eines Abstandes zwischen Kanüle und Gefäßwänden.

Erfindungsgemäß weist die expandierte Spitze Öffnungen auf, die das Vorbeiströmen eines Teil des Blutes ermöglichen. Vorteilhafterweise kann daher der Anteil des Blutes, der nicht durch die Kanüle und dadurch aus dem Körper heraus fließt, bei geringfügigem Durchflusswiderstand die Kanüle umfließen.

In einer bevorzugten Ausführungsform ist bevorzugt, wenn die expandierbare Struktur eine selbstexpandierbare Struktur ist.

Bei dieser Ausführungsform besitzt also die Vorrichtung im sog. Ruhezustand, d.h. expandierten Zustand, eine bereits expandierte Struktur an ihrem distalen Ende, wobei durch Ausübung einer Kraft auf die expandierte Struktur diese in einen zusammengedrückten, komprimierten Zustand überführt werden kann. In diesem komprimierten Zustand kann die Vorrichtung in das gewünschte Gefäß eingeführt werden, wobei nach Platzierung in dem Gefäß durch Kraftentlastung die Struktur wieder in den expandierten Zustand übergeht. Dieser Übergang in die expandierte Form wird in vergleichbaren Fachgebieten als selbstexpandierbar bezeichnet.

Diese Maßnahme hat den Vorteil, dass die Kanüle, insbesondere die Spitze bzw. das distale Ende der Kanüle, bereits in der expandierten Konfiguration hergestellt werden kann, die somit ihrem Ruhezustand entspricht. Vor und während der Einführung in das Gefäß kann das distale Ende mit der expandierbaren Struktur derart druck- bzw. kraftbelastet werden, dass die expandierbare Struktur aufgrund der Komprimierung in eine langgestreckte Form gedehnt wird, wodurch sich das distale Ende im Vergleich zum restlichen Durchmesser der Kanüle verjüngt. Wird der Druck bzw. die Kraft nach erfolgter Einführung in das Gefäß entfernt, kehrt die expandierbare Struktur des distalen Endes wieder in ihre ursprüngliche expandierte Konfiguration zurück. Soll die Kanüle nach erfolgter Anwendung wieder entfernt werden, wird die expandierbare Struktur des distalen Endes wieder durch Kraft in den komprimierten, sich in Längsrichtung zur Kanüle erstreckenden Zustand überführt, wodurch sich die Spitze bzw. das distale Ende wiederum verjüngt und einfach aus dem Gefäß entfernt werden kann.

In einer anderen Ausführungsform kann bevorzugt sein, wenn die expandierbare Struktur über bspw. Kraft-/Druckeinwirkung in den expandierbaren Zustand überführbar ist. Dies kann bspw. durch einen inflatierbaren Ballon erfolgen, der innerhalb der expandierbaren Struktur vorgesehen ist. Der sich ausbreitende Ballon überträgt dabei die Kraft auf die expandierbare Struktur, wodurch diese in den expandierten Zustand überführt wird.

Insgesamt ist bevorzugt, wenn die expandierbare Struktur ein Material aufweist, das ausgewählt ist aus der Gruppe umfassend elastische Kunststoffe, superelastische Kunststoffe, elastische Metalle, superelastische Metalle, insbesondere Nitinol.

In einer weiteren Ausführungsform ist bevorzugt, wenn die expandierbare Struktur ein Material aufweist, das Shape-Memory-Eigenschaften besitzt.

Dem Fachmann wird anhand einer Anzahl im Stand der Technik bekannten Materialien klar sein, welches Material gewählt werden kann bzw. muss, wobei sich im Stand der Technik insbesondere Nitinol als geeignetes Material herausgestellt hat.

In einer weiteren Ausführungsform ist bevorzugt, wenn die expandierbare Struktur den Aufbau eines selbstexpandierbaren Flechtstents besitzt.

Selbstexpandierbare Flechtstents sind im Stand der Technik hinreichend bekannt und beschrieben. Diese stellen in einem bestimmten Winkel geflochtene Drahtkonstrukte dar, die sich nach Entfernen einer komprimierenden Hülle selbst entfalten.

Je nach dem spezifischen Winkel, in dem der Stent geflochten ist, lassen sich die Öffnungen in der expandierbaren Struktur einstellen. Diese Struktur kann sich gemäß dem bevorzugten Beispiel am distalen Kanülenende der erfindungsgemäßen Vorrichtung befinden und bildet dann die expandierbare Struktur.

In einer weiteren Ausführungsform der erfindungsgemäßen Vorrichtung ist bevorzugt, wenn die Kanüle ein drahtverstärktes, stentartiges Grundgerüst aufweist, wobei das proximale Ende und der Zwischenabschnitt zur Ausbildung eines Flüssigkeitsdichten Röhrenabschnitts mit einem Kunststoff beschichtet sind.

Diese Ausführungsform hat den Vorteil, dass die Drahtstruktur der Kanüle entlang ihrer Länge Stabilität verleiht, wobei gleichzeitig das distale Ende frei von dem elastischen Kunststoff ist und daher Öffnungen aufweist, deren Größe und Form durch den Flechtwinkel des drahtverstärkten Grundgerüsts vorgegebenen wird; ferner kann sich das distale Ende, da es nicht mit einem Stabilität verleihenden Kunststoff beschichtet ist, nach Druckentlastung expandieren, wodurch die Öffnungen freigegeben werden.

In einer weiteren bevorzugten Ausführungsform ist bevorzugt, wenn der Winkel, mit dem das Drahtgrundgerüst geflochten wird, über dessen Länge variabel ist.

Durch diese Maßnahme kann in unterschiedlichen Bereichen des Grundgerüsts - und letztendlich auch der Kanüle - ein unterschiedlicher Winkel erreicht werden, mit dem sich wiederum die Größe der Öffnungen regulieren lässt.

In einer weiteren bevorzugten Ausführungsform ist der Kunststoff, mit dem die Vorrichtung gemäß der Erfindung zumindest teilweise beschichtet werden kann, ausgewählt aus der Gruppe umfassend Polyurethan, Silicon und Teflon.

Danach wird bei dem Ausführungsbeispiel zunächst bspw. ein geflochtener Drahtstent bereitgestellt, der entweder über seine gesamte Länge, oder aber lediglich in bestimmten Abschnitten einen ganz bestimmten Flechtwinkel aufweist. Dieses "Grundgerüst" wird anschließend in seinem proximalen Ende und in seinem Zwischenabschnitt mit einem Kunststoff beschichtet, das distale Ende hingegen bleibt frei von der Kunststoffbeschichtung. Das proximale Ende und der Zwischenabschnitt bilden daher ein dichtes Röhrchen, durch das bspw. Blut geleitet werden kann, während das distale Ende unbeschichtet ist und die Struktur eines Flechtstents aufweist. Dieser bildet daher im expandierten Zustand am distalen Ende Öffnungen, die durch den Winkel der sich schneidenden/überlappenden Drähte des Flechtstents gebildet werden. Durch das expandierte Ende kann die Kanüle durch die Anlagerung der Drähte an die Gefäßwand einerseits im Gefäß verankert werden; andererseits kann durch die Öffnungen Blut an der Kanüle vorbei fließen und nicht nur in diese hinein, wodurch - bei einer arteriellen Kanülierung - die Versorgung der stromabwärts gelegenen Körperbereiche eines Patienten gewährleistet ist, und - bei einer venösen Kanülierung - der physiologische Rückfluss zum Herzen gewährleistet ist und venöse Blutstauungen bzw. Thrombosen vermieden werden.

Bei einer weiteren Ausführungsform weist die Vorrichtung ferner einen Dilator mit einem proximalen und einem distalen Bereich sowie mit einem zwischen proximalen und distalen Bereich gelegenen Zwischenabschnitt auf, wobei der Dilator zur Einführung der Kanüle innerhalb dieser vorgesehen ist.

Ein Dilator ist ein üblicher Vorrichtungsbestandteil von Kanülen. Mit einem Dilator werden die Gefäße gedehnt und dadurch für die Einführung der Kanüle und deren Festsetzung im Gefäß vorbereitet. Vorliegend wird also bei der erfindungsgemäßen Vorrichtung der Dilator in die Kanüle eingeführt, um das Einführen der Kanüle in ein Gefäß zu erleichtern, und bildet damit einen integralen Bestandteil der Vorrichtung.

Bei einer Ausführungsform der erfindungsgemäßen Vorrichtung ist bevorzugt, wenn durch den Dilator die expandierbare Struktur ihrer Länge nach verschiebbar ist.

Diese Ausführungsform hat den Vorteil, dass der Dilator, der ohnehin oft Bestandteil eines Kanülierungssystems ist, zur Einführung der Kanüle in deren nicht-expandierten Zustand eingesetzt werden kann. Der Dilator wird hierbei vor der Einführung der Kanüle in das Gefäß in die Kanüle eingeführt und verkantet sich an dem distalen Ende der Kanüle mit der expandierbaren Struktur, wodurch bei einem weiteren Vorschieben des Dilators in die Kanüle die expandierbare Struktur verschoben, bzw. in die Länge gezogen wird. Durch dieses Verschieben wird der ursprüngliche Durchmesser der expandierbaren Struktur verringert.

Ferner ist allgemein bevorzugt, wenn die expandierbare Struktur durch Entfernen eines Dilators, der innerhalb der Vorrichtung zur Einführung der Kanüle in ein Gefäß geführt wird, in ihren expandierten Zustand überführbar ist.

Der Dilator wird dabei zur Einführung der Vorrichtung innerhalb der Kanüle geführt, und ermöglicht beim Durchgang durch die Haut und durch die Gefäßwand die Stabilität der Kanüle und dient der Erweiterung des Gefäßes. Der Dilator kann vorliegend die notwendige Kraft erbringen, um die expandierbare Struktur des distalen Endes in den nicht-expandierten Zustand, bzw. in die langgestreckte Form zu bringen.

Dies kann bspw. durch eine besondere Ausführung des distalen Bereiches des Dilators erfolgen, wonach der Dilator in diesem Bereich an seiner äußeren Oberfläche Strukturen aufweist, die sich bei Einführung des Dilators in die Kanüle mit der expandierenden Struktur verkanten, und bei einem weiteren Vorschieben in Richtung distal die expandierbare Struktur in die Länge strecken. Bei einem Rückziehen des Dilators entkantet sich die expandierbare Struktur von den Strukturen der Oberfläche des Dilators, wodurch dieser durch die Kanüle und aus dem Gefäß herausgeführt werden kann und die expandierbare Struktur des distalen Endes der Kanüle in deren expandierten Zustand überführt wird.

Diese Maßnahme kann bspw. dadurch erreicht werden, dass die expandierbare Struktur am distalen Ende der Vorrichtung die Form eines auf den Zwischenabschnitt der Kanüle aufgesetzten kugelförmigen, drahtverstärkten Grundgerüsts aufweist. Das Ende dieses Grundgerüsts, welches sich zum Gefäß öffnet, ist dabei verjüngt und weist einen kleineren Durchmesser auf als ein distaler Bereich des Dilators. Aufgrund der Verwendung eines elastischen, vorzugsweise superelastischen, Materials für die expandierbare Struktur - also dem Grundgerüst - und im Zusammenspiel mit dem kleineren Durchmesser des Grundgerüsts nimmt der Dilator bei dessen Einführung in die Kanüle beim Auftreffen auf die verjüngte Struktur diese in Schiebrichtung mit, wodurch die expandierbare Struktur in die Länge gezogen wird. Der Umfang der Längenausdehnung der expandierbaren Struktur hängt von dem verwendeten Material der expandierbaren Struktur und ggf. dem Flechtwinkel ab, wenn ein geflochtenes Grundgerüst verwendet wird.

Andererseits kann aber auch eine flexible Hülle oder ein hüllartiger Abschnitt vorgesehen sein, der zur Einführung der Vorrichtung in ein Gefäß zumindest über die expandierbare Struktur geführt ist, und der die expandierbare Struktur damit vor und während der Einführung der Vorrichtung im nicht-expandierten Zustand hält. Nach korrekter Platzierung der Kanüle kann dann die Hülle - bspw. durch Zurückziehen in Richtung Anwender - entfernt werden, wodurch die expandierbare Struktur freigegeben wird und sich expandieren kann.

Ein weiteres Merkmal der erfindungsgemäßen Vorrichtungen ist das variable Lumen der Kanülen. Die Kanülenspitze, also das distale Ende, die sich im Gefäß befindet, weist zur Einbringung der Kanüle einen niedrigeren Durchmesser auf als der proximale Teil und/oder der Zwischenabschnitt der Kanüle. Dadurch werden die Verletzungsgefahr und die Störung der Strömungsverhältnisse außerhalb der Kanüle reduziert. Im Gegensatz zu den im Stand der Technik bekannten Kanülen ist es daher nicht notwendig, die Kanüle weit in das Gefäß einzuschieben, um die Lage der Kanüle auch durch einen relativ breiten Durchmesser zu sichern. Mit der expandierbaren Struktur wird gewährleistet, dass zumindest ein Abstand zwischen Gefäßwänden und Kanüle gesichert ist. Die Kanüle kann also nur zu einem geringen Teil mit ihrem distalen Ende in das Gefäß eingeführt werden, wobei das distale Ende beim Einführen in das Gefäß einen geringeren Durchmesser aufweist, wodurch Gefäßverletzungen vermieden werden. Gleichzeitig weist die Kanüle in dem Teil, der außerhalb des Gefäßes liegt, d.h. im proximalen Ende, einen größeren Durchmesser auf, wodurch der gesamte Widerstand zum Blutdurchfluss in der Kanüle abnimmt. Die Durchmesservergrößerung stellt nun keine Verletzungsgefahr mehr dar, weil das Gewebe außerhalb des Gefäßes ohne negative Folgen ausgedehnt werden kann.

Ferner ist von Vorteil, dass der dünne Teil der Kanüle, also das distale Ende, innerhalb des Gefäßes wesentlich kürzer ist als der breitere Teil außerhalb des Gefäßes, wodurch sich ein insgesamt sehr kleiner Widerstand ergibt.

Die genaue Einführung der Kanüle in das Gefäß bis zur gewünschten Länge kann auf verschiedene Weisen erfolgen.

In einer weiteren Ausführungsform ist bevorzugt, wenn der Dilator eine kanalartige Passage aufweist, der sich vom distalen Bereich des Dilators bis zumindest dem Zwischenabschnitt des Dilators erstreckt.

Die Passage kann sich dabei entweder als kanalartige Vertiefung auf der Außenoberfläche des Dilators befinden, oder aber als vollständig im Dilator vorgesehener Kanal.

Insbesondere ist bevorzugt, wenn die kanalartige Passage als längliche Vertiefung auf der Oberfläche des Dilators vorgesehen ist, wobei ein Ende der Passage als Einlass im distalen Bereich des Dilators vorgesehen ist und wobei das andere Ende der Passage als Auslass im proximalen Ende oder im Zwischenabschnitt vorgesehen ist.

Diese Ausführungsform hat den Vorteil, dass durch die kanalförmige Passage die Gefäßseite mit der Anwenderseite verbunden werden kann. Dies bedeutet, dass, wenn die Kanüle mit dem Dilator in das Gefäß eingeschoben wird, und sich das distale Ende von sowohl der Kanüle als auch dem Dilator innerhalb des Gefäßes befindet, das sich in dem Gefäß befindende Blut wegen der herrschenden Kapillarkräfte und des Blutdrucks in die kanalförmige Vertiefung fließt. Über die kanalförmige Vertiefung, die ihren Beginn, d.h. den Einlass, am distalen Ende besitzt, wird das Blut dann mindestens bis zum Zwischenabshcnitt des Dilators geführt, wo es durch Austritt aus dem Auslass der kanalförmigen Passage für den Anwender sichtbar wird. Dadurch wird für den Anwender sichtbar, dass die Positionierung der Vorrichtung erfolgreich war, und dass in einem nächsten Schritt die Kanüle durch Entfernen des Dilators freigesetzt werden kann.

Bei einer weiteren Ausführungsform ist bevorzugt, wenn der Einlass und der Auslass der kanalartigen Passage jeweils mit einer membranartigen Abdeckung versehen sind. Insbesondere ist bevorzugt, wenn die kanalartige Passage des Dilators ein fließfähiges oder gasförmiges Medium, wie bspw. Luft, Stickstoff, Kochsalzlösung, aufweist.

Diese Maßnahme hat den Vorteil, dass der Nachweis des erfolgreichen Gefäßzuganges auch ohne Blutdurchgang durch die Passage des Dilators erfolgen kann.

Die Ausführungsform mit der membranartigen Abdeckung hat den Vorteil, dass, wenn sich die Kanüle im Gefäß befindet, die Membran über dem Einlass der kanalartigen Passage im Gefäß von dem Blutdruck gedehnt und nach innen gedrückt wird. Wenn am anderen Ende der kanalartigen Passage, bzw. am Auslass, eine weitere Membran die Passage abdichtet, so wölbt sich diese aufgrund des erhöhten Drucks in der Passage auf.

Dabei kann bei einer bevorzugten Ausführungsform vorgesehen sein, dass sich in der Passage Gas befindet, welches vorzugsweise kompressibel ist.

Ferner kann in der Passage auch eine Flüssigkeit vorhanden sein, so dass bei erfolgter Gefäßzugangslegung eine Membranwölbung auf der Anwenderseite durch die Volumenverdrängung der Flüssigkeit in der Passage bewirkt wird. In diesem Fall kann die Flüssigkeit beispielsweise eine Kochsalzlösung sein, die entweder bereits im Dilator vorhanden ist, oder aber vom Anwender vor Gebrauch eingeführt wird.

In einer weiteren Ausführungsform ist bevorzugt, wenn die Membran des Dilators an der Gefäßseite eine wesentlich größere Fläche als die Membran an der Anwenderseite aufweist.

Diese Maßnahme hat den Vorteil, dass eine kleine Wölbung der menbranartigen Abdeckung an der Blutseite zu einer größeren Wölbung an der Anwenderseite, und somit zu einem eindeutigen Signal für den Anwender, führt.

Insgesamt ist bevorzugt, wenn zumindest Teile der erfindungsgemäßen Vorrichtung mit zumindest einem biokompatiblen Material beschichtet sind. Ein solches Material wird gewählt, um die Blutgerinnungsgefahr und Entzündungsreaktionen, die durch Kontakt der Gefäße und des Blutes mit Fremdmaterial ausgelöst werden, zu vermindern, wenn nicht sogar um diese zu verhindern. Verschiedene natürliche und/oder synthetische Materialien, die für den beanspruchten Einsatz geeignet sind, sind dem Fachmann aus dem Stand der Technik bekannt.

In einer weiteren Ausführungsform ist bevorzugt, wenn die Kanüle über ihre Länge einen variablen Durchmesser besitzt.

Diese Maßnahme hat den Vorteil, dass bspw. das distale Ende der Kanüle zur Einführung der Kanüle in ein Gefäß einen kleineren Durchmesser aufweisen kann als das proximale Ende, bzw. der Zwischenabschnitt der Kanüle. Entsprechend kann der in die Kanüle einzubringende Dilator der Form und damit den unterschiedlichen Durchmessern einer Kanüle angepasst sein.

Die vorliegende Erfindung betrifft ferner die Verwendung der Vorrichtung in einem extrakorporalen Kreislaufsystem.

Bei solchen Verwendungen kann die erfindungsgemäße Vorrichtung bspw. im Zusammenhang mit einem Lung-Assist-Device in der extrakorporalen Lungenunterstützung eingesetzt werden. Mit der neuen Vorrichtung wird die Blutversorgung von unterhalb der Kanülierungsstelle gelegenen Bereichen sichergestellt.

Mit der vorliegenden Erfindung wird eine Vorrichtung zur Kanülierung eines Blut-führenden Gefäßes bereitgestellt, durch welche mittels der schmalen und expandierbaren Kanülenspitze ein schneller und schonender Gefäßzugang unter variablen anatomischen und physiologischen Bedingungen auch bei Noteinsätzen ermöglicht wird. Mit der erfindungsgemäßen Vorrichtung wird die Verletzungs- und Überdehnungsgefahr, die bei großen Kanülen besteht, wesentlich verringert. Ferner wird ein Verschluss des Peripheriegefäßes durch die Kanüle über die Expandierung des distalen Endes verhindert, so dass auch die Körperextremitäten mit physiologischen Blutflussraten durchblutet werden. Ferner wird die Durchflussrate innerhalb der Kanüle unter physiologischen Bedingungen maximiert. Der Strömungswiderstand im kleinen Kanülendurchmesser innerhalb des Gefäßes wird durch den kleinen Widerstand im großen Durchmesser außerhalb der Kanüle kompensiert. Der resultierende Kanülenwiderstand ist damit insgesamt kleiner als bei traditionellen Kanülen, obwohl die Blutströmung zu den Extremitäten, bzw. der venöse Blutrückstrom aus den Extremitäten zum Herz nicht beeinträchtigt wird.

Weitere Vorteile ergeben sich aus den beigefügten Figuren sowie der nachstehenden Beschreibung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: einen Ausschnitt einer Ausführungsform der erfindungsgemäßen Vorrichtung, in dem das distale Ende mit der expandierbaren Struktur gezeigt ist;
- Fig. 2a: einen Ausschnitt einer Ausführungsform der erfindungsgemäßen Vorrichtung, nämlich das distale Ende im Querschnitt, im kraftbelasteten Zustand der expandierbaren Struktur;
- Fig. 2b: den Ausschnitt aus Fig. 2a, wobei sich hier die expandierbare Struktur durch Kraftentlastung im expandierten Zustand befindet;
- Fig. 3: einen Ausschnitt einer weiteren Ausführungsform der erfindungsgemäßen Vorrichtung;
- Fig. 4a: ein Kanülierungsschema aus dem Stand der Technik;
- Fig. 4b: das Einbringen einer Ausführungsform der erfindungsgemäßen Vorrichtung in ein Blut-führendes Gefäß;
- Fig. 5: eine Ausführungsform der erfindungsgemäßen Vorrichtung, bei der der Dilator eine Passage aufweist;
- Fig. 6a: das distale Ende einer weiteren Ausführungsform der Vorrichtung, die eine membranartige Abdeckung auf der Passage aufweist;
- Fig. 6b: die membranartige Abdeckung der Ausführungsform aus Fig. 5a für den Ausgang am proximalen Ende der Vorrichtung der Passage.

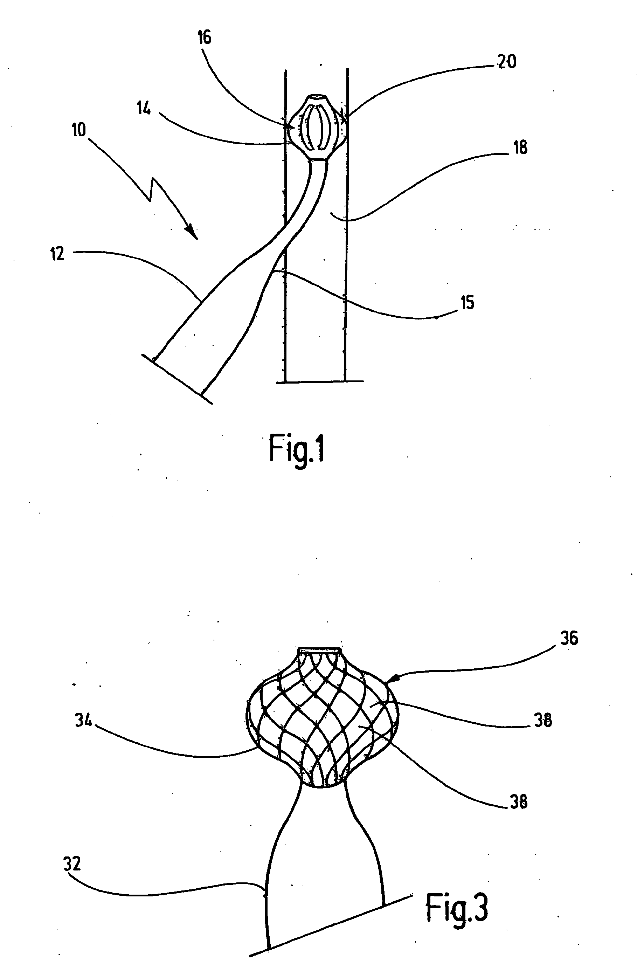

In Fig. 1 ist mit 10 insgesamt ein Ausschnitt einer Ausführungsform der erfindungsgemäßen Vorrichtung zur Kanülierung eines Blut-führenden Gefäßes gezeigt. Die Vorrichtung 10 weist eine Kanüle 12 auf mit einem proximalen Ende, einem distalen Ende 14 sowie einem Zwischenabschnitt 15 auf, der zwischen proximalen Ende und distalem Ende 14 liegt. Das distale Ende 14 weist eine expandierbare Struktur 16 auf, die im Gefäß 18 im expandierten Zustand vorliegt. Ferner sind mit 20 Öffnungen in der expandierbaren Struktur 16 gezeigt. Die Kanüle 12 weist ferner eine Kanülenöffnung auf, über die die Fluidverbindung des Gefäßes mit der Vorrichtung hergestellt wird

Wie Fig. 1 zu entnehmen ist, wurde das distale Ende 14 der Kanüle 12 in das Gefäß 18 eingeführt. In Fig. 1 hat sich die expandierbare Struktur 16 des distalen Endes 14 im expandierten Zustand an die Gefäßwände angelegt. Andererseits muss die expandierbare Struktur sich nicht an die Gefäßwand anlegen, sie sichert lediglich durch ihre Form einen Abstand der Kanüle von den Gefäßwänden. Dadurch sichert sie den Sitz der Kanüle 12 bzw. der Vorrichtung 10 insgesamt in dem Gefäß. Nach Einführen der Vorrichtung in das Gefäß 18 gelangt somit ein Teil des in dem Gefäß 18 geführten Blutes über die Kanülenöffnung in die Kanüle 12 und anschließend außerhalb des Körpers eines Patienten, bspw. in einen extrakorporalen Kreislauf wie in eine extrakorporale Lungenunterstützung mit einem Lung-Assist-Device. Der andere Teil des Blutes, der nicht in die Kanüle 12 geführt wird, strömt durch die Öffnungen 20 an der Kanüle vorbei und gelangt in Gefäßabschnitte unterhalb der Kanülierungsstelle. Damit ist die Blutversorgung dieser Gefäßabschnitte, und nicht zuletzt der Körperextremitäten, gewährleistet.

Die Öffnungen der expandierbaren Struktur können jede beliebige Form einnehmen, insbesondere solche, die strömungstechnisch geeignet sind, also solche, die ein günstiges Aufteilungs-Verhältnis zwischen dem über die Kanüle 12 abzuführendem Blut und dem im Gefäß 18 weiterzuleitendem Blut ermöglichen.

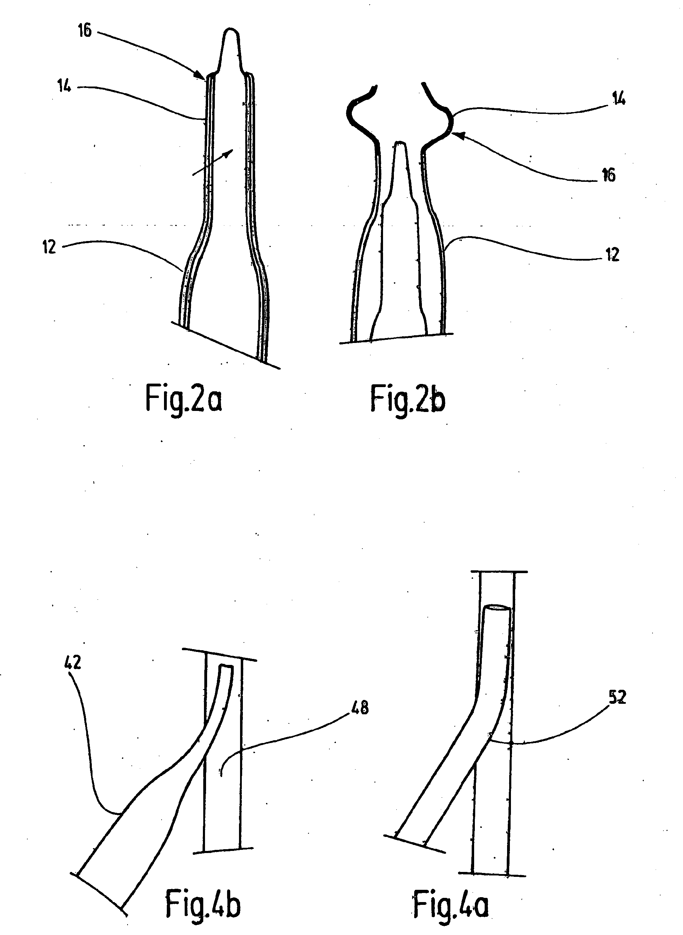

In Fig. 2a und 2b sind wiederum Abschnitte einer Ausführungsform der erfindungsgemäßen Vorrichtung gezeigt, wobei gleiche Elemente wie in Fig. 1 mit den gleichen Bezugszeichen versehen wurden. In Fig. 2a und 2b ist das distale Ende 14 der Kanüle 12 mit der expandierbaren Struktur 16 insgesamt im Längsschnitt gezeigt, wobei sich in der Kanüle 12 ferner ein Dilator 22 befindet.

Der Dilator kann ein üblicherweise in diesem Gebiet verwendeter Dilator darstellen, und Materialien wie Kunststoffe aufweisen. Dilatoren dienen, wie bereits weiter oben erwähnt, im Allgemeinen der Dehnung der Gefäße, in die die Kanülen eingebracht werden sollen.

In Fig. 2a befindet sich die expandierbare Struktur in ihrem nicht-expandierten Zustand. Ferner ist der Dilator 22 durch die Kanüle 12 geführt, der sich auch durch die expandierbare Struktur 16 hindurch erstreckt und diese dadurch entlang der Längsachse des Dilators streckt, bzw. verschiebt, und dadurch den Querschnitt der expandierbaren Struktur, den sie im expandierten Zustand besitzt, verkleinert. Die Verschiebung kann bspw. durch ein Verkanten des Dilators mit der expandierbaren Struktur erreicht werden. In diesem Zustand wird folglich auf die expandierbare Struktur 16 eine Kraft ausgeübt, mit der sie bis zur erfolgreichen Platzierung der Kanüle 12 im Gefäß 18 gehalten wird.

In Fig. 2b ist der expandierte Zustand der expandierbaren Struktur 16 des distalen Enden 14 der Kanüle 12 gezeigt, in den sie nach Platzierung der Kanüle 12 im Gefäß 18 überführt wird. Diesen Zustand erreicht sie durch Entfernen des Dilators 22 und konsequenter Kraftentlastung. Bei diesem Ausführungsbeispiel weist die expandierbare Struktur ein Material mit superelastischer Eigenschaft auf, bzw. ist aus diesem hergestellt, die es ermöglicht, dass die expandierbare Struktur in expandierter Form hergestellt, zur Einführung in ein Gefäß zusammengedrückt, und nach Platzierung der Kanüle 12 im Gefäß 18 und nach Entfernen der auf sie ausgeübten Kraft ihre ursprüngliche Form wiederhergestellt werden kann.

In Fig. 3 ist ein Ausschnitt einer weiteren Ausführungsform der erfindungsgemäßen Vorrichtung dargestellt, wobei das distale Ende 34 der Kanüle 32 gezeigt ist, welches eine expandierbare Struktur 36 aufweist. Wie der Fig. 3 zu entnehmen ist weist die expandierbare Struktur 36 die Gestalt einer geflochtenen Drahtstruktur nach Art eines Flechtstents auf. Die in Fig. 3 gezeigte Ausführungsform befindet sich im expandierten Zustand. Wie Fig. 3 zu entnehmen ist weist die expandierbare Struktur 36 Öffnungen 38 auf, die nach Platzieren der Kanüle 32 in einem Gefäß gewährleisten, dass ein Teil des in dem Gefäß geführten Blutes an der Kanüle vorbei in unterhalb der Kanülierungsstelle gelegene Bereiche, insbesondere in die Körperextremitäten, geführt wird.

Erfindungsgemäß kann die Vorrichtung als Grundgerüst eine geflochtene Drahtstruktur aufweisen, deren proximales Ende sowie der Zwischenabschnitt mit einem Kunststoff beschichtet sind. Durch die Beschichtung dieser Abschnitte wird ein flüssigkeitsdichter, röhrchenförmiger Kanülenabschnitt geschaffen, der an seinem distalen Ende noch den Aufbau und die Form der geflochtenen Struktur aufweist. Das Grundgerüst kann dabei über seine Länge unterschiedliche Flechtwinkel aufweisen.

Andererseits kann die Vorrichtung auch derart aufgebaut sein, dass die expandierbare Struktur separat hergestellt und mit den weiteren Abschnitten der Kanüle fest verbunden wird.

In den Fig. 4a und 4b ist ein prinzipieller Unterschied zwischen Kanülen des Standes der Technik und Ausführungsformen der erfindungsgemäßen Vorrichtung dargestellt: Die im Stand der Technik bekannten Kanülen 52, gezeigt in Fig. 4a, weisen einen konstanten Durchmesser auf, wohingegen die erfindungsgemäße Vorrichtungschematisch dargestellt in Fig. 4b - ein variables Lumen aufweisen kann: Die Kanüle 42 besitzt innerhalb des Gefäßes 48 einen niedrigeren Durchmesser, wodurch die Verletzungsgefahr und die Störung der Strömungsverhältnisse außerhalb der Kanüle reduziert wird. Gleichzeitig weist die Kanüle außerhalb des Gefäßes 48 einen größeren Durchmesser auf als der Abschnitt der Kanüle, der sich in dem Gefäß befindet, wodurch der gesamte Widerstand zum Blutdurchfluss in der Kanüle abnimmt. Da der dünne Teil der Kanüle 42 innerhalb des Gefäßes 48 wesentlich kürzer ist als der Teil mit dem größeren Durchschnitt außerhalb des Gefäßes 48, ergibt sich insgesamt ein sehr kleiner Widerstand. Die Möglichkeit, den Teil der Kanüle, der sich innerhalb des Gefäßes 48 befindet, kurz zu halten, wird durch die expandierbare Struktur - in Fig. 4b nicht gezeigt - ermöglicht. Erst diese Struktur ermöglicht bei dieser Ausführungsform der erfindungsgemäßen Vorrichtung die Verankerung der Kanüle in einem Gefäß, so dass es nicht notwendig ist, längere Abschnitte in die Gefäße einzuführen, um eine sichere Platzierung zu erhalten.

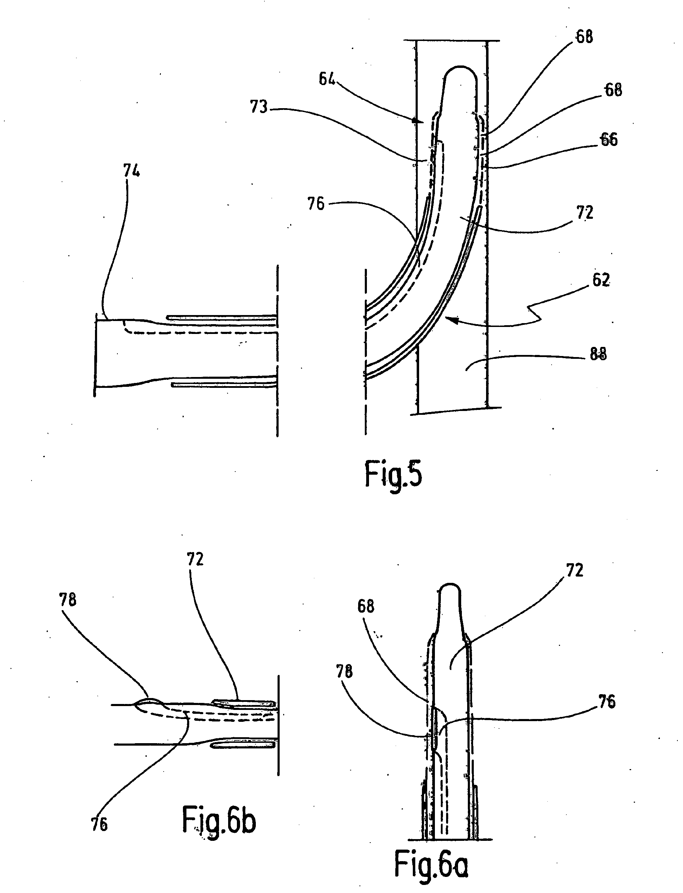

In Fig. 5 ist ein Ausschnitt einer anderen Ausführungsform der erfindungsgemäßen Vorrichtung dargestellt. In Fig. 5 ist mit 62 insgesamt die Kanüle bezeichnet, sowie mit 64 das distale Ende der Kanüle 62. Die expandierbare Struktur ist mit 66 gekennzeichnet, die Öffnungen der expandierbaren Struktur 66 mit 68. Ferner ist in Fig. 5 zu erkennen, dass die Kanüle 62 mit Hilfe eines Dilators 72 in ein Gefäß 88 eingeführt wurde. Der Dilator 72 weist eine Passage 76 auf, die in Fig. 5 mit gestrichelten Linien gekennzeichnet ist. Die Passage 76 befindet sich an einer Oberfläche des Dilators 72 und erstreckt sich ausgehend von dem distalen Abschnitt 73 des Dilators 72 bis zumindest in einen Zwischenabschnitt 74 des Dilators 72. Durch die Einbringung des Dilators 72 in die Kanüle 62 und damit auch in die expandierbare Struktur 66, wird diese in Längsrichtung des Dilators 72 verschoben. Durch die in der expandierbaren Struktur 66 vorgesehenen Öffnungen 68 gelangt Blut in die Passage 76. Da der Zwischenabschnitt der Kanüle 62 keine Öffnungen aufweist, schließt die Kanüle die Passage 76 in deren weiteren Verlauf in Längsrichtung dicht ab, so dass das durch die Öffnungen 68 in die Passage 76 eingeflossene Blut durch die Passage 76 bis zu deren Ausgang geführt wird, der sich im Zwischenabschnitt oder am proximalen Ende des Dilators 72 befindet, jedenfalls aber in dem Bereich des Dilators 72, der sich nicht mehr innerhalb der Kanüle befindet. Sobald daher nach Einbringung der Kanüle 62 in ein Gefäß Blut am Ausgang der Passage 76 auftritt, kann der Anwender der Vorrichtung erkennen, dass die Kanüle weit genug eingebracht wurde.

In den Fig. 6a und 6b sind Ausschnitte einer weiteren Ausführungsform der erfindungsgemäßen Vorrichtung gezeigt. In den Fig. 6a und 6b wurden gleiche Bezugszeichen wie in Fig. 5 verwendet, sofern gleiche Elemente beschrieben werden. In den Fig. 6a und 6b ist zu erkennen, dass die Passage 76 an ihrem Einlass (siehe Fig. 6b) im distalen Bereich des Dilators 72 sowie an ihrem Auslass (siehe Fig. 6a) mit einer membranartigen Abdeckung 78 bedeckt ist. Bei dieser Ausführungsform ist die Passage mit einem Medium, bspw. Kochsalzlösung, gefüllt. Wird die Kanüle in ein Gefäß eingeführt, so wird die Membran, sobald sie in Kontakt mit dem fließenden Blut kommt, an dieser Stelle eingedrückt. Durch Druckübertragung auf das in der Passage 76 vorliegende Medium wird die Membran am Auslass gewölbt, wodurch der Anwender wiederum erkennen kann, dass die Kanüle das Gefäß erreicht hat.

## Patentansprüche

1. Vorrichtung (10) zur Kanülierung eines Hohlorgans, insbesondere eines arteriellen oder venösen Blut-führenden Gefäßes (48; 88), mit einer Kanüle (12; 32, 42, 62), die ein proximales Ende, ein distales Ende (14; 34, 64) und einen zwischen dem proximalen und dem distalen Ende gelegenen Zwischenabschnitt (15) aufweist, wobei die Kanüle (12; 32, 42, 62) nach Einbringung in das Gefäß in Fluidverbindung mit dem Gefäß steht, und wobei das distale Ende (14; 34, 64) eine expandierbare Struktur (16; 36, 66) aufweist, die von einem nicht-expandierten Zustand in einen expandierten Zustand überführbar ist, wobei die expandierbare Struktur (16; 36, 66) zumindest im expandierten Zustand Öffnungen (20; 38, 68) aufweist, **dadurch gekennzeichnet, dass** die expandierbare Struktur (16; 36, 66) den Aufbau eines selbstexpandierbaren Flecht-Stents besitzt und dass die Kanüle (12; 32, 42, 62) ein drahtverstärktes, flechtstentartiges Grundgerüst aufweist, wobei das proximale Ende und der Zwischenabschnitt zur Ausbildung eines flüssigkeitsdichten Röhrenabschnitts mit einem Kunststoff beschichtet sind.

2. Vorrichtung (10) nach Anspruch 1, **dadurch gekennzeichnet, dass** die expandierbare Struktur (16; 36, 66) eine selbstexpandierende Struktur ist.

3. Vorrichtung (10) nach einem der Ansprüche 1 oder 2, **dadurch gekennzeichnet, dass** die expandierbare Struktur (16; 36, 66) ein Material aufweist, das ausgewählt ist aus der Gruppe umfassend, elastische Kunststoffe, superelastische Kunststoffe, elastische Metalle, superelastische Metalle.

4. Vorrichtung (10) nach einem der Ansprüche 1 bis 3, **dadurch gekennzeichnet, dass** die expandierbare Struktur (16; 36, 66) ein Material aufweist, das Shape-Memory Eigenschaften besitzt.

5. Vorrichtung (10) nach Anspruch 3 oder 4, **dadurch gekennzeichnet, dass** das Material Nitinol ist.

6. Vorrichtung (10) nach Anspruch 5, **dadurch gekennzeichnet, dass** das flechtstentartige Grundgerüst über seine Länge variable Winkel aufweist.

7. Vorrichtung (10) nach einem der Ansprüche 1 bis 6, **dadurch gekennzeichnet, dass** sie ferner einen Dilator (22; 72) mit einem proximalen und einem distalen Bereich sowie mit einem zwischen proximalen und distalen Bereich gelegenen Zwischenabschnitt aufweist, wobei der Dilator zur Einführung der Kanüle innerhalb dieser vorgesehen ist.

8. Vorrichtung (10) nach Anspruch 7, **dadurch gekennzeichnet, dass** durch den Dilator (22; 72) die expandierbare Struktur ihrer Länge nach verschiebbar ist.

9. Vorrichtung (10) nach einem der Ansprüche 7 oder 8, **dadurch gekennzeichnet, dass** der Dilator (72) eine kanalartige Passage (76) aufweist, die sich vom distalen Bereich (73) des Dilators bis zumindest dem Zwischenabschnitt (74) des Dilators (72) erstreckt.

10. Vorrichtung (10) nach Anspruch 9, **dadurch gekennzeichnet, dass** die kanalartige Passage als längliche Vertiefung auf der Oberfläche des Dilators vorgesehen ist, wobei ein Ende der Passage als Einlass im distalen Bereich des Dilators vorgesehen ist und wobei das andere Ende der Passage als Auslass im proximalen Ende oder im Zwischenabschnitt vorgesehen ist.

11. Vorrichtung (10) nach Anspruch 10, **dadurch gekennzeichnet, dass** der Einlass und der Auslass jeweils mit einer membranartigen Abdeckung (78) versehen sind.

12. Vorrichtung (10) nach Anspruch 11, **dadurch gekennzeichnet, dass** die Passage (76) ein fließfähiges oder gasförmiges Medium aufweist.

13. Vorrichtung (10) nach einem der Ansprüche 1 bis 12, **dadurch gekennzeichnet, dass** die Kanüle (12; 32; 42; 62) über ihre Länge variable Durchmesser aufweist.

## Claims

1. Device (10) for cannulisation of a hollow organ, in particular an arterial or venous blood-carrying vessel (48; 88), with a cannula (12; 32, 42, 62), a proximal end, a distal end (14; 34, 64) and a central section (15) arranged between the proximal end and distal end, wherein said cannula (12; 32, 42, 62) after insertion into the vessel is in fluid communication with the vessel, and wherein the distal end (14; 34, 64) has an expandable structure (16; 36, 66) which can be converted from a non-expanded state to an expanded state, and wherein the expandable structure (16; 36, 66) at least in the expanded state comprises openings (20; 38, 68), **characterised in that** the expandable structure (16; 36, 66) has the construction of a self-expandable braided stent and the cannula (12; 32, 42, 62) has a wire-reinforced, braided stent-like main frame, wherein the proximal end and the central section are coated with plastic to form a liquid-tight tube section.

2. Device (10) according to claim 1, **characterised in that** the expandable structure (16; 36, 66) is a self-expanding structure.

3. Device (10) according to claims 1 or 2, **characterised in that** the expandable structure (16; 36, 66) has a material chosen from the group comprising elastic plastics, superelastic plastics, elastic metals, superelastic metals.

4. Device (10) according to claims 1 to 3, **characterised in that**, the expandable structure (16; 36, 66) has a material exhibiting shape-memory properties.

5. Device (10) according to claims 3 or 4, **characterised in that** the material is Nitinol.

6. Device (10) according to claim 5, **characterised in that** the braided stent-like main frame has variable angles along its length.

7. Device (10) according to claim 1 to 6, further **characterised in that** it further comprises a dilator (22; 72) with a proximal area and a distal area and a central section located between the proximal and distal areas, wherein the dilator is provided to introduce the cannula to within the section between the proximal and distal areas.

8. Device (10) according to claim 7, **characterised in that** the expandable structure can be displaced along its length through the dilator (22; 72).

9. Device (10) according to claims 7 or 8, **characterised in that** the dilator (72) has a channel-like passage (76) that extends from the distal area (73) of the dilator to at least the central section (74) of the dilator (72).

10. Device (10) according to claim 9, **characterised in that** the channel-like passage is provided as an elongate depression on the upper surface of the dilator, wherein one end of the passage being provided as an inlet in the distal area of the dilator, and wherein the other end of the passage being provided as an outlet in the proximal end or in the central section.

11. Device (10) according to claim 10, **characterised in that** the inlet and the outlet are each provided with a membrane-like cover (78).

12. Device (10) according to claim 11, **characterised in that** the passage (76) has a free-flowing or gaseous medium.

13. Device (10) according to claims 1 to 12, **characterised in that** the cannula (12; 32; 42; 62) has variable diameters along its length.

## Revendications

1. Dispositif (10) pour le canulage d'un organe creux, en particulier un vaisseau (48 ; 88) sanguin artériel ou veineux, comportant une canule (12 ; 32, 42, 62) qui possède une extrémité proximale, une extrémité distale (14 ; 34, 64) et un tronçon intermédiaire (15) situé entre l'extrémité proximale et l'extrémité distale, la canule (12 ; 32, 42, 62) étant en liaison fluidique avec le vaisseau après son introduction dans ledit vaisseau, et l'extrémité distale (14 ; 34, 64) étant constituée d'une structure expansible (16 ; 36, 66), qui peut être amenée d'un état non déployé à un état déployé, ladite structure expansible (16 ; 36, 66) comportant des ouvertures (20 ; 38, 68) au moins à l'état déployé, **caractérisé en ce que** la structure expansible (16 ; 36, 66) possède la structure d'un stent tressé auto-expansible, et **en ce que** la canule (12 ; 32, 42, 62) comporte une structure de base en forme de stent tressé, renforcée par du fil, l'extrémité proximale et le tronçon intermédiaire étant revêtus d'une matière plastique pour réaliser une partie tubulaire étanche aux liquides.

2. Dispositif (10) selon la revendication 1, **caractérisé en ce que** la structure expansible (16 ; 36, 66) est une structure auto-expansible.

3. Dispositif (10) selon la revendication 1 ou 2, **caractérisé en ce que** la structure expansible (16 ; 36, 66) comporte un matériau qui est choisi dans le groupe contenant des matières plastiques élastiques, des matières plastiques superélastiques, des métaux élastiques, des métaux superélastiques.

4. Dispositif (10) selon l'une quelconque des revendications 1 à 3, **caractérisé en ce que** la structure expansible (16 ; 36, 66) comporte un matériau qui possède des propriétés de mémoire de forme.

5. Dispositif (10) selon la revendication 3 ou 4, **caractérisé en ce que** le matériau est le nitinol.

6. Dispositif (10) selon la revendication 5, **caractérisé en ce que** la structure de base en forme de stent tressé présente un angle variable sur sa longueur.

7. Dispositif (10) selon l'une quelconque des revendications 1 à 6, **caractérisé en ce qu'**il comporte, en outre, un dilatateur (22 ; 72) muni d'une zone proximale et d'une zone distale, ainsi que d'une zone intermédiaire située entre la zone proximale et la zone distale, le dilatateur étant prévu à l'intérieur de la canule pour l'introduction de cette dernière.

8. Dispositif (10) selon la revendication 7, **caractérisé en ce que** la structure expansible peut coulisser en longueur à travers le dilatateur (22 ; 72).

9. Dispositif (10) selon la revendication 7 ou 8, **caractérisé en ce que** le dilatateur (72) comporte un passage (76) en forme de conduit, qui s'étend depuis la zone distale (73) du dilatateur au moins jusqu'à la zone intermédiaire (74) du dilatateur (72).

10. Dispositif (10) selon la revendication 9, **caractérisé en ce que** le passage en forme de conduit est prévu sous la forme d'un creux allongé sur la surface du dilatateur, une extrémité du passage étant prévue comme entrée dans la zone distale du dilatateur et l'autre extrémité du passage étant prévue comme sortie dans l'extrémité proximale ou dans la zone intermédiaire.

11. Dispositif (10) selon la revendication 10, **caractérisé en ce que** l'entrée et la sortie sont munies chacune d'un cache (78) formé par une membrane.

12. Dispositif (10) selon la revendication 11, **caractérisé en ce que** le passage (76) conduit un fluide liquide ou gazeux.

13. Dispositif (10) selon l'une quelconque des revendications 1 à 12, **caractérisé en ce que** la canule (12 ; 32, 42, 62) présente un diamètre variable sur sa longueur.
